# EUROPÄISCHE PATENTSCHRIFT

(11) **EP 2 509 506 B1**
(45) Veröffentlichungstag und Bekanntmachung des Hinweises auf die Patenterteilung: **08.02.2017**
(21) Anmeldenummer: 10796318.3
(22) Anmeldetag: 08.12.2010
(51) Int. Cl.: A61B 6/00, A61B 6/10

(54) **STRAHLENSCHUTZVORRICHTUNG**
RADIATION PROTECTION DEVICE
DISPOSITIF DE RADIOPROTECTION

(30) Priorität: 08.12.2009 DE 102009057366
(43) Veröffentlichungstag der Anmeldung: 17.10.2012
(73) Patentinhaber: Mavig GmbH, 81829 München (DE)
(72) Erfinder: JESCHKE, Richard, 85221 Dachau (DE)
(74) Vertreter: Vossius & Partner Patentanwälte Rechtsanwälte mbB
(86) Internationale Anmeldenummer: PCT/EP2010/069184
(87) Internationale Veröffentlichungsnummer: WO 2011/070076

(56) Entgegenhaltungen:
- EP-A1- 0 146 055
- WO-A1-2004/107979
- US-A- 3 984 696
- US-A- 4 923 162
- US-A- 5 006 718
- US-A- 5 981 964

## Beschreibung

Die vorliegende Erfindung betrifft eine Strahlenschutzvorrichtung, insbesondere eine Schutzvorrichtung zur Abschirmung der durch eine Röntgenstrahlungsquelle abgestrahlten Röntgenstrahlung, welche beispielsweise zur Verwendung an einem angiographischen Arbeitsplatz vorgesehen ist.

Um die durch Röntgenuntersuchungen hervorgerufenen Strahlenbelastungen für die beteiligten Personen möglichst niedrig zu halten, ist es bereits seit langem bekannt, Strahlenschutzbekleidung zu verwenden. Diese Strahlenschutzbekleidungen bieten zwar in bestimmten Fällen einen sehr effektiven Schutz gegen eine überhöhte Strahlenbelastung, an sogenannten angiographischen Arbeitsplätzen ist der damit erzielbare Schutz allerdings nur unzureichend. Die Strahlenexposition eines Arztes an einem derartigen Arbeitsplatz durch die multidirektionale Strahlenrichtung ist besonders hoch, so dass zusätzlich zum Tragen einer Röntgenschutzbekleidung eine sogenannte Unterkörperschutzanordnung verwendet wird.

Eine Unterkörperschutzanordnung die an einem Tisch montierbar und klappbar ist, ist aus US3984696 bekannt.

Eine solche Unterkörperschutzanordnung ist auch aus der EP 1 613 217 B1 bekannt.

Demnach besteht eine Unterkörperschutzanordnung, welche seitlich an einem Patiententisch angeordnet werden kann, aus einer Abschirmmatte in Form einer in PVC eingeschichteten Bleigummimatte oder Bleifolie mit einem Bleigleichwert von 0,5 mm, die von der Tischebene des Patiententisches bis zu dem Fußboden reicht und die von der Röntgenschutzkleidung nicht bedeckten unteren Extremitäten vor Streustrahlung schützt. Dabei wird mit dem Bleigleichwert das Absorptionsverhalten eines Körpers, insbesondere eines Laminates beschrieben, das gegenüber Röntgenstrahlung eine Abschirmung aufweist, wie sie eine Bleiplatte der entsprechenden Dicke hat.

Die aus der EP 1 613 217 B1 bekannte Unterkörperschutzanordnung weist mehrere PVC-Bleigummi-Lamellen auf, die seitlich nebeneinander und zumindest teilweise überlappend angeordnet sind. Die Lamellen sind an einer Trägerschiene befestigt, an der zusätzlich ein Oberteil angeordnet werden kann, das zusätzlichen Schutz oberhalb des Patiententisches bietet.

Das Oberteil ist dabei als Aufsatz ausgebildet, der sich an der Trägerschiene befestigen lässt und von dieser wieder abnehmen lässt.

Es ist ein Nachteil dieser bekannten Unterkörperschutzanordnung, dass das nach oben über dem Patiententisch hervorstehende Oberteil häufig den Zugang zum Patienten auf dem Patiententisch erschwert bzw. den Patiententisch blockiert. Da dieser Aufsatz häufig im Wege ist und nur dann benötigt wird, wenn tatsächlich Röntgenstrahlung emittiert wird, muss der Aufsatz häufig abgenommen und wieder aufgesetzt werden. Dieses Prozedere erschwert den Arbeitsablauf im Operationssaal, da u.a. der abgenommene Aufsatz irgendwo abgelegt werden muss. Ferner wird durch das Abnehmen des Aufsatzes die Trägerschiene zusammen mit deren Befestigungsvorrichtungen für den Aufsatz bloßgelegt, an der bzw. an denen sich ein Patient verletzen könnte.

Es ist demnach eine Aufgabe der vorliegenden Erfindung, eine Strahlenschutzvorrichtung bereit zu stellen, die den oben genannten Problemen Rechnung trägt. Es ist insbesondere eine Aufgabe der vorliegenden Erfindung, eine effektive Strahlenschutzvorrichtung bereitzustellen, die zum einen sicheren Schutz gewährt und zum anderen den Arbeitsablauf während einer Untersuchung nicht behindert oder erschwert. Diese Aufgabe wird durch eine Strahlenschutzvorrichtung nach Anspruch 1 gelöst.

Erfindungsgemäß wird eine Strahlenschutzvorrichtung zum Anbringen an einem Patiententisch bereit gestellt. Die Strahlenschutzvorrichtung weist einen Träger und ein an dem Träger vorgesehenes Abschirmelement auf, wobei das Abschirmelement mittels mindestens eines Doppelgelenks derart am Träger montiert ist, dass das Abschirmelement zumindest zwischen einer hochgeklappten Stellung und einer heruntergeklappten Stellung beweglich ist. In der hochgeklappten Stellung bietet das Abschirmelement hinreichenden Schutz gegen Streustrahlung, die von einem Patienten ausgeht, der auf dem Patiententisch liegt. Wird dieser Schutz gerade nicht benötigt, weil etwa das Röntgengerät nicht emittiert, kann das Abschirmelement auf einfache Weise in eine heruntergeklappte Stellung überführt werden. In dieser heruntergeklappten Stellung ist das Abschirmelement derart am Patiententisch angeordnet, dass es den Zugang zum Patiententisch bzw. zu einem auf diesem liegenden Patienten nicht behindert.

Es ist ferner bevorzugt, dass der Träger durch das Abschirmelement in der heruntergeklappten Stellung im Wesentlichen abgedeckt ist. Hierzu kann das Abschirmelement eine optional flexible Verlängerung aufweisen, die nicht notwendigerweise strahlenundurchlässig zu sein braucht. Auf diese Weise kann sich der Patient nicht an dem Träger oder an an diesem vorgesehenen Befestigungsvorrichtungen verletzen. Ferner wird dadurch der Träger sowie die an diesem vorgesehene Mechanik vor Verschmutzung geschützt.

Vorzugsweise weist das Abschirmelement der erfindungsgemäßen Strahlenschutzvorrichtung genau zwei stabile Stellungen, nämlich die hochgeklappte und die heruntergeklappte Stellung, auf, in denen es fixierbar ist. Gemäß einer alternativen Variante kann das Abschirmelement in mehreren oder beliebig vielen Stellungen zwischen der hochgeklappten Stellung und der heruntergeklappten Stellung fixiert werden.

Das Doppelgelenk ist derart am Träger angebracht, dass in der hochgeklappten Stellung des Abschirmelementes mindestens eine Schwenkachse des Doppelgelenks blockiert ist. Auf diese Weise wird das Abschirmelement in der hochgeklappten Stellung durch die Blockierung der Doppelgelenkachse fixiert, ohne dass es einer zusätzlichen Fixierungseinrichtung bedürfte.

Das Abschirmelement ist zumindest in der hockgeklappten Stellung höhenverstellbar. Dadurch lässt sich das Abschirmelement an eine gegebene Situation, beispielsweise die Ausmaße eines Patienten, anpassen. Weiterhin ist das Abschirmelement zumindest in der hochgeklappten Stellung schwenkbar und/oder neigbar ist. Auch dies dient der Optimierung des Schutzes je nach Situation.

Gemäß einer besonders vorteilhaften Ausführungsform der vorliegenden Erfindung ist das Doppelgelenk als Doppelgelenkbolzen ausgebildet. Auch wenn sich die vorliegende Erfindung mit einem einzelnen Doppelgelenkbolzen realisieren lässt, ist es bevorzugt, wenn das Abschirmelement mittels mindestens zweier Doppelgelenkbolzen am Träger montiert ist. Dabei ist es bevorzugt, dass die mindestens zwei Doppelgelenkbolzen durch dafür vorgesehene Durchgangsöffnungen im Träger geführt sind. Die unterschiedlichen Stellungen des Abschirmelementes können dann durch Verschieben der Doppelgelenkbolzen in den Durchgangsöffnungen des Trägers realisiert werden.

Bevorzugt weisen die Doppelgelenkbolzen drei jeweils gelenkig miteinander verbundene Bolzenabschnitte auf. Dabei ist die Länge eines ersten Bolzenendabschnittes größer als die Dicke des Trägers und die Länge eines zweiten Bolzenendabschnittes vorzugsweise kleiner als die Dicke des Trägers. Ferner weist ein mittlerer Bolzenabschnitt bevorzugt eine Länge auf, die größer ist als der Abstand der Durchgangsöffnungen im Träger vom Rand des Trägers. Dies ermöglicht im Wesentlichen eine Faltung des Doppelgelenkbolzens um den Träger.

Bevorzugt ermöglichen die beiden Gelenke des Doppelgelenks jeweils einen Schwenkwinkel im Bereich zwischen 70° und 110°. Mit anderen Worten können im Falle des Doppelgelenkbolzens die gelenkig miteinander verbundenen Bolzenabschnitte jeweils der Länge nach ausgerichtet sein bzw. einen im Wesentlichen senkrechten Winkel zueinander einnehmen.

Gemäß einer bevorzugten Ausführungsform der erfindungsgemäßen Strahlenschutzvorrichtung ist am Träger ferner mindestens ein Untertischstrahlenschutzelement lösbar anbringbar. Vorzugsweise sind hierfür mehrere Untertischstrahlenschutzelemente nebeneinander und überlappend angeordnet. Das Untertischstrahlenschutzelement ist dabei vorzugsweise schwingbar aufgehängt. Das Abschirmelement der Strahlenschutzvorrichtung ist vorzugsweise im oberen Bereich abgewinkelt.

Nachfolgend werden bevorzugte Ausführungsformen der erfindungsgemäßen Strahlenschutzvorrichtung anhand der Figuren näher erläutert. Es zeigen:
- Fig. 1: einen angiographischen Arbeitsplatz mit einer Strahlenschutzanordnung gemäß dem Stand der Technik;
- Fig. 2: eine bevorzugte Ausführungsform der erfindungsgemäßen Strahlenschutzvorrichtung in Vorderansicht;
- Fig. 3: eine bevorzugte Ausführungsform der erfindungsgemäßen Strahlenschutzvorrichtung in Seitenansicht;
- Fig. 4: ein Detail der Fig. 3;
- Fig. 5: die Strahlenschutzvorrichtung der Fig. 3 ohne Abschirmelement in Rückansicht;
- Fig. 6: einen in der erfindungsgemäßen Strahlenschutzvorrichtung zum Einsatz kommenden Doppelgelenkbolzen;
- Fig. 7: einen Bolzenendabschnitt des Doppelgelenkbolzens der Fig. 6;
- Fig. 8: einen Schnitt entlang C-C in Fig. 6;
- Fig. 9: eine schematische Darstellung des Doppelgelenkbolzens in einer Führungsöffnung eines Trägers in der hochgeklappten Stellung;
- Fig. 10: eine schematische Darstellung des Doppelgelenkbolzens in der Führungsöffnung des Trägers in der heruntergeklappten Stellung;
- Fig. 11: eine Seitenansicht des Trägers; und
- Fig. 12: eine bevorzugte Ausführungsform der erfindungsgemäßen Strahlenschutzvorrichtung in heruntergeklappter Stellung.

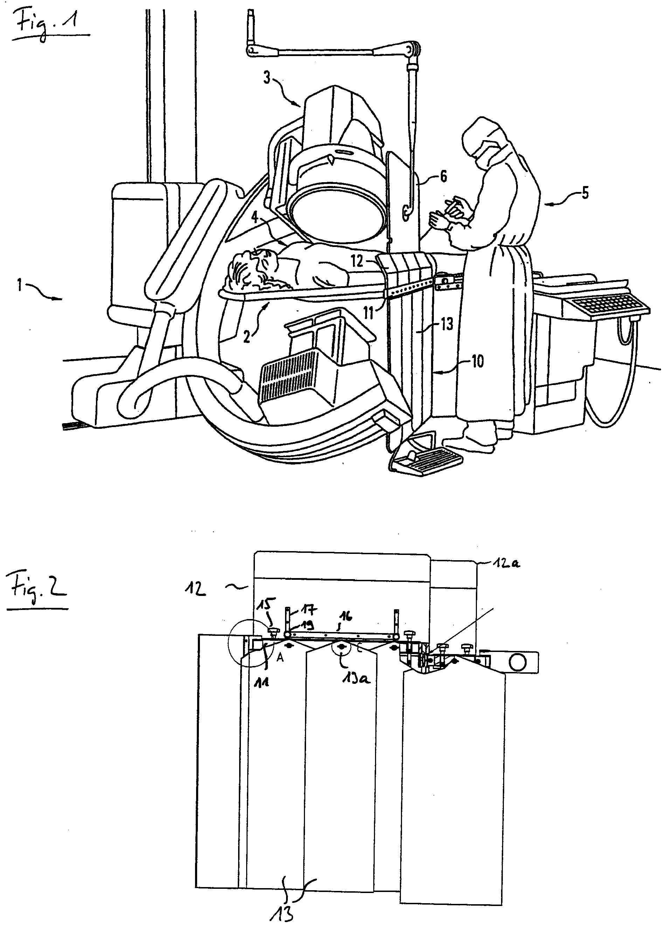

Fig. 1 zeigt einen allgemein mit dem Bezugszeichen 1 versehenen angiographischen Arbeitsplatz, X dessen wesentliche Bestandteile ein höhenverstellbarer Patiententisch 2 sowie eine Röntgenanordnung 3 sind. Die Röntgenanordnung 3 ist schwenkbar gelagert, um eine möglichst flexible Ausrichtung des Röntgengenerators auf den Patienten 4 zu gewährleisten.
Als Folge hiervon ergibt sich, dass Röntgenstrahlung und die entsprechenden Streustrahlen in verschiedensten Richtungen austreten können.

Um daher eine an dem Arbeitsplatz 1 tätige Person 5 möglichst umfangreich vor diesen Strahlen schützen zu können, sind neben der Strahlenschutzbekleidung der Person 5 zusätzliche Strahlenschutzmaßnahmen vorgesehen. Im vorliegenden Fall bestehen diese aus einer Strahlenschutzscheibe 6, die eine Abschirmung des Oberkörpers des behandelnden Arztes 5 sowie dessen Kopfes ermöglichen soll. Darüber hinaus ist eine Unterkörperschutzanordnung 10 vorgesehen, die beispielsweise aus der EP 1 613 217 B1 bekannt ist. Diese Unterkörperschutzanordnung 10, welche am seitlichen Bereich des Behandlungstisches 2 befestigt ist, besteht aus einem Oberteil 12, das an einer an dem Tisch 2 befestigten Trägerschiene 11 angeordnet ist, sowie aus mehreren an der Unterseite der Trägerschiene 11 befestigten Lamellen 13, die seitlich nebeneinander überlappend angeordnet sind. Die überlappende Anordnung der Lamellen 13 hat eine besonders hohe Flexibilität der Anordnung zufolge, welche einen sehr wirksamen Strahlenschutz ermöglicht.

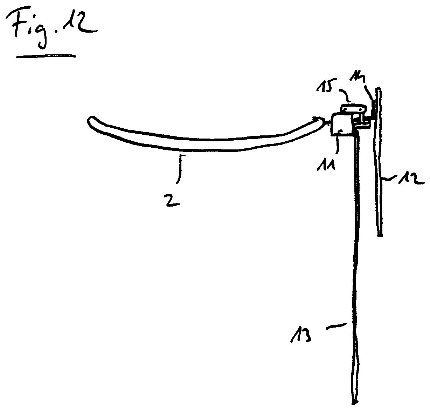

Während die Strahlenschutzscheibe 6 mit einem einfachen Handgriff verschoben werden kann, lässt sich das Oberteil 12, das beispielsweise im Weg ist, wenn der Patient 4 auf dem Patiententisch 2 gelagert werden soll, lediglich komplett abnehmen. Dies ist relativ umständlich und erschwert das Arbeiten an einem solchen angiographischen Arbeitsplatz 1. Um diesem Problem Rechnung zu tragen, ist es daher erfindungsgemäß vorgesehen, dass anstelle des bekannten Oberteils 12 ein Abschirmelement vorgesehen ist, welches mittels mindestens eines Doppelgelenks derart an einem Träger bzw. der Trägerschiene 11 montiert ist, dass das Abschirmelement zumindest zwischen einer hochgeklappten Stellung entsprechend der Fig. 2 und einer heruntergeklappten Stellung entsprechend der Fig. 12 beweglich ist. Auf diese Weise lässt sich das Abschirmelement mit einem einfachen Handgriff nach unten klappen, was dann einen erleichterten Zugang zum Patiententisch 2 bzw. zum Patienten 4 ermöglicht. Während in Fig. 12 durch das Herunterklappen lediglich ein Zugang zum Patiententisch 2 geschaffen wurde, kann zusätzlich an dem Abschirmelement 12 eine nicht dargestellte, vorzugsweise flexible Verlängerung vorgesehen sein, die im heruntergeklappten Zustand den Träger 11 und den Doppelgelenkbolzen 15 abdeckt und somit diese Teile vor Verschmutzung und den Patienten vor Verletzung schützt.

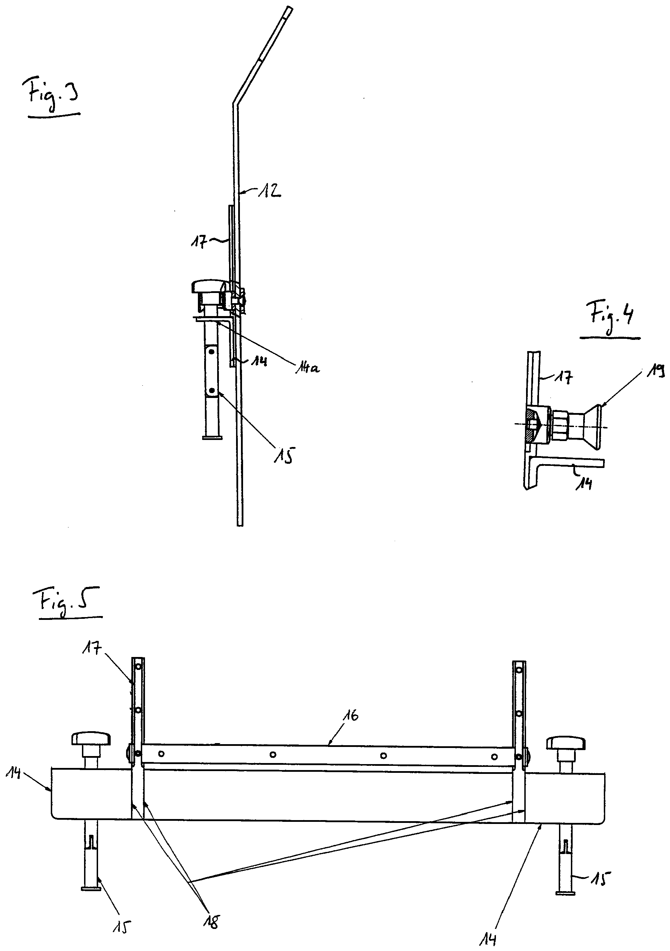

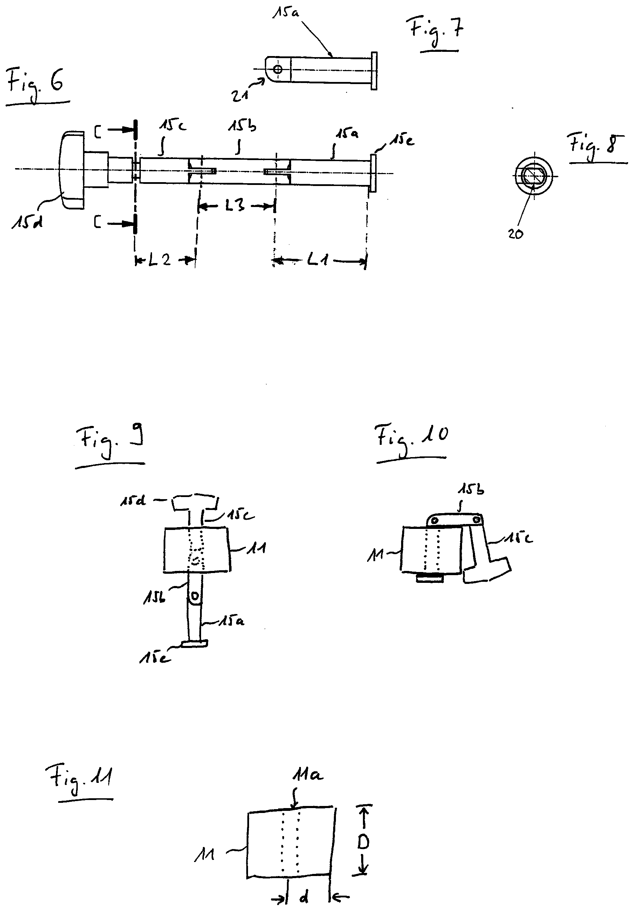

In Fig. 3 ist eine Seitenansicht einer erfindungsgemäßen Strahlenschutzvorrichtung ohne Träger bzw. Trägerschiene dargestellt. Die Strahlenschutzvorrichtung weist im Wesentlichen ein Abschirmelement 12 auf, das mittels eines Winkels 14 mit zwei Doppelgelenkbolzen 15 verbunden ist. Das Abschirmelement ist vorzugsweise in seinem oberen Bereich abgewinkelt ausgebildet. Bevorzugt ist der abgewinkelte Bereich des Abschirmelements 12 zum Patienten geneigt, um Strahlung vom Patienten verstärkt abzuschirmen. Die Trägerschiene 11 (in Fig. 3 nicht dargestellt) weist zwei entsprechende Führungsöffnungen 11a (vgl. Fig. 11) auf, die die Doppelgelenkbolzen 15 aufnehmen. Durch Verschieben der Doppelgelenkbolzen 15 in den Führungsöffnungen 11 a des Trägers bzw. der Trägerschiene 11 kann das Abschirmelement 12 der erfindungsgemäßen Strahlenschutzvorrichtung zwischen einer hochgeklappten Stellung und einer heruntergeklappten Stellung bewegt werden, wie anhand der schematischen Darstellung gemäß den Figuren 9 und 10 veranschaulicht ist.

Der Doppelgelenkbolzen 15 weist einen ersten Bolzenendabschnitt 15a, einen zweiten Bolzenendabschnitt 15c sowie einen mit diesen beiden gelenkig verbundenen mittleren Bolzenabschnitt 15b auf. Am Ende des ersten Bolzenendabschnittes 15a ist ein Vorsprung 15e vorgesehen. Der zweite Bolzenendabschnitt 15c geht in einen Griffabschnitt 15d über. Der Doppelgelenkbolzen 15 wird dabei so in der Durchgangsöffnung 11a des Trägers 11 (vgl. Fig. 11) geführt, dass er zwischen einer ersten in Fig. 9 dargestellten und einer zweiten in Fig. 10 dargestellten Stellung verschieblich ist. In der in Fig. 9 dargestellten Situation ist der Doppelgelenkbolzen 15 im Wesentlichen vertikal im Träger 11 gelagert. Dadurch wird das an dem Doppelgelenkbolzen 15 befestigte Abschirmelement 12 in der in Fig. 3 dargestellten hochgeklappten Stellung gehalten.

Durch Ziehen an den Griffabschnitten 15d der Doppelgelenkbolzen 15 können diese aus der in Fig. 9 dargestellten Position nach oben gezogen werden. Eine solche Bewegung wird durch die an den ersten Bolzenendabschnitten 15a vorgesehenen Vorsprünge 15e begrenzt bzw. gestoppt. Dann können der mittlere Bolzenabschnitt 15b und der zweite Bolzenendabschnitt 15c so abgewinkelt werden, dass der Doppelgelenkbolzen 15 entsprechend der Fig. 10 im Wesentlichen um den Träger 11 herum gefaltet wird. Dadurch wird das an dem Doppelgelenkbolzen 15 befestigte Abschirmelement 12 aus der in Fig. 3 dargestellten hochgeklappten Stellung in eine heruntergeklappte Stellung überführt, wodurch ein Zugang zum Patiententisch bzw. Patienten geschaffen wird. In Fig. 12 ist schematisch ein erfindungsgemäßes Abschirmelement 12 in der heruntergeklappten Stellung gezeigt.

Für diese Funktionalität ist es besonders vorteilhaft, wenn der Doppelgelenkbolzen 15 bestimmte Maße aufweist. Wie in Fig. 6 dargestellt, haben die ersten und zweiten Bolzenendabschnitte 15a und 15c jeweils eine Länge L1 bzw. L2 und der mittlere Bolzenabschnitt 15b eine Länge L3. Der Träger 11 hat, wie in Fig. 11 skizziert, eine Dicke D, wobei der Abstand der Durchgangsöffnung 11a vom Rand des Trägers d beträgt. Für das Umklappen gemäß Fig. 10 ist es von Vorteil, wenn die Länge L1 des ersten Bolzenendabschnittes 15a größer ist als die Dicke D des Trägers 11 und die Länge L3 des mittleren Bolzenabschnittes 15b größer ist als der Abstand d der Durchgangsöffnung vom Rand des Trägers 11. Es ist ferner bevorzugt, dass die Länge L2 des zweiten Bolzenendabschnittes 15c kleiner ist als die Dicke D des Trägers 11. Auf diese Weise wird das Gelenk, das den mittleren Bolzenabschnitt 15b mit dem zweiten Bolzenendabschnitt 15c verbindet, in der in Fig. 9 dargestellten Situation durch die Führungsöffnung 11 a des Trägers 11 blockiert.

Da das Abschirmelement 12 aus der hochgeklappten Stellung (vgl. Fig. 3) im Wesentlichen nur in eine Richtung, nämlich vom Patiententisch weg, geklappt werden soll, ist es ferner bevorzugt, dass die beiden Gelenke des Doppelgelenkbolzens jeweils Schwenkwinkel im Bereich zwischen 70° und 110°, besonders bevorzugt von etwa 90° ermöglichen. Mit anderen Worten, soll der Doppelgelenkbolzen 15 aus der im Wesentlichen gestreckten Stellung lediglich in einer Richtung an jedem Gelenk um etwa 90° geklappt werden können (vgl. Fig. 10). Hierzu sind die gelenkbildenden Enden 21 der Bolzenendabschnitte 15a und 15c wie in Fig. 7 dargestellt, asymmetrisch geformt, so dass ein Abknicken der aneinandergrenzenden Bolzenabschnitte im Wesentlichen nur in eine Richtung möglich ist. Um eine entsprechende Ausrichtung des Gelenkbolzens 15 bzgl. des Abschirmelementes 12 zu gewährleisten, ist der zweite Bolzenendabschnitt 15c abschnittsweise mit einem Querschnitt versehen, der wie in Fig. 8 zu sehen beispielsweise zwei im Wesentlichen parallele Seitenflächen aufweist. Durch eine entsprechende Öffnung 14a im Winkelement 14 (vgl. Fig. 3), in der der Doppelgelenkbolzen 15 aufgenommen ist, wird ein Verdrehen des Doppelgelenkbolzens 15 in der Öffnung 14a verhindert.

In einer bevorzugten Ausführungsform ist das Abschirmelement über eine Führungsschiene 17 mit dem Winkel 14 verbunden. In Fig. 5 sind zwei vertikale Führungsschienen 17 zu sehen, die direkt mit dem Winkel 14 verschweißt sind, wie mit dem Bezugszeichen 18 angedeutet. Ferner ist eine waagerechte Führung 16 vorgesehen. Die waagerechte Führung 16 kann an den vertikalen Führungen nach oben bzw. unten gleiten und mittels Einrastbolzen 19 (vgl. Fig. 4) an diesen fixiert werden. Dadurch ist das Abschirmelement 12 in der hochgeklappten Stellung zusätzlich höhenverstellbar. Anstelle der separaten Öffnungen für den Einrastbolzen 19 in den Führungsschienen 17 können alternativ in den Führungsschienen 17 auch Langlöcher vorgesehen sein. Dadurch kann das Abschirmelement 12 stufenlos höhenverstellt werden. Die Langlöcher können zusätzlich eine Kulissenführung aufweisen, so dass das Abschirmelement 12 zusätzlich gedreht bzw. gekippt werden kann.

In Fig. 2 ist eine erfindungsgemäße Strahlenschutzvorrichtung in Vorderansicht, d.h. von der Arztseite aus gesehen, dargestellt. Das Abschirmelement 12 ist hier mittels der Doppelgelenkbolzen 15, die in dem Träger 11 fixiert sind, in der hochgeklappten Stellung montiert. Zu sehen sind ferner die waagerechte Führungsschiene 16 sowie die vertikalen Führungsschienen 17. Mit Hilfe der Einrastbolzen 19 lässt sich demnach auf einfache Weise von der Arztseite aus die Höhe des Abschirmelementes 12 in der hochgeklappten Stellung anpassen. Soll das Abschirmelement 12 aus der in Fig. 2 dargestellten Stellung heruntergeklappt werden, muss der Arzt lediglich die beiden Doppelgelenkbolzen 15 nach oben ziehen und das Abschirmelement 12 nach vorne, d.h. zu sich, klappen lassen. Nach diesem einfachen Handgriff hat der Arzt freien Zugang zum Patienten, ohne durch das Abschirmelement 12 behindert zu sein, wie in Fig. 12 zu sehen ist.

Wie in Fig. 2 dargestellt, kann zusätzlich zu dem Abschirmelement 12 ein weiteres Abschirmelement 12a vorgesehen sein, das auf analoge Weise angebracht und ebenso nach unten geklappt werden kann. Ferner können mehrere an der Unterseite der Trägerschiene 11 angeordnete Lamellen 13 vorgesehen sein, die seitlich nebeneinander überlappend angeordnet sind. Erfindungsgemäß werden die einzelnen Lamellen 13 jeweils mit Hilfe von Befestigungseinrichtungen 13a an der Trägerschiene 11 angehängt. Besonders bevorzugt ist eine schwingbare Aufhängung. Eine Anbringung, die eine Befestigung bzw. ein Lösen der Lamellen ohne Werkzeug ermöglicht, ist hierfür bevorzugt. Dies kann beispielsweise mit Hilfe von Kugelrastbolzen oder mittels Druckknöpfen geschehen. Alternativ ist auch eine Schlüssellochfixierung oder ein Drehverschluss möglich, wie er üblicherweise bei Planen von LKWs angewandt wird.

Sowohl die Schutzlamellen 13 als auch die Abschirmelemente 12 und 12a weisen vorzugsweise einen Bleigleichwert von 0,5 mm auf. Das Abschirmelement sowie die Schutzelemente können beispielsweise eine in PVC eingeschichtete Bleigummimatte oder Bleifolie aufweisen. Es sind aber auch andere Materialien verwendbar, die einen entsprechenden Schutz gewähren.

Die Strahlenschutzvorrichtung der vorliegenden Erfindung gewährt einerseits einen effektiven Schutz gegen Röntgenstrahlung und sonstige Strahlen und ermöglicht andererseits einen reibungslosen Arbeitsablauf, in dem sie mit einem einfachen Handgriff weggeklappt werden kann. Dadurch wird dem Arzt der Zugriff auf den Patienten wesentlich erleichtert.

## Patentansprüche

1. Strahlenschutzvorrichtung (10) zur Anbringung an einem Patiententisch (2) wobei die Vorrichtung einen Träger (11) und ein an dem Träger (11) vorgesehenes Abschirmelement (12) aufweist, wobei das Abschirmelement (12) mittels mindestens eines Doppelgelenks (15) derart am Träger (11) montiert ist, dass das Abschirmelement (12) zumindest zwischen einer hochgeklappten Stellung und einer heruntergeklappten Stellung beweglich ist; wobei das Abschirmelement (12) zumindest in der hochgeklappten Stellung höhenverstellbar und schwenkbar und/oder neigbar ist; und wobei das Doppelgelenk (15) derart am Träger (11) angebracht ist, dass in der hochgeklappten Stellung mindestens eine Schwenkachse des Doppelgelenks (15) blockiert ist.

2. Strahlenschutzvorrichtung (10) nach Anspruch 1, wobei das Doppelgelenk (15) als Doppelgelenkbolzen (15) ausgebildet ist.

3. Strahlenschutzvorrichtung (10) nach Anspruch 2, wobei der mindestens eine Doppelgelenkbolzen (15) durch eine Durchgangsöffnung (11a) im Träger (11) geführt ist.

4. Strahlenschutzvorrichtung (10) nach Anspruch 3, wobei der mindestens eine Doppelgelenkbolzen (15) drei gelenkig miteinander verbundene Bolzenabschnitte aufweist, wobei ein erster Bolzenendabschnitt (15a) eine Länge (L1) aufweist, die größer als die Dicke (D) des Trägers (11) ist, wobei ein mittlerer Bolzenabschnitt (15b) vorzugsweise eine Länge (L3) aufweist, die größer als der Abstand (d) der Durchgangsöffnung vom Trägerrand ist, und wobei ein zweiter Bolzenendabschnitt (15c) bevorzugt eine Länge (L2) aufweist, die kleiner als die Dicke (D) des Trägers (11) ist.

5. Strahlenschutzvorrichtung (10) nach einem der Ansprüche 1 bis 4, wobei die beiden Gelenke des mindestens einen Doppelgelenks (15) jeweils Schwenkwinkel im Bereich zwischen 70° und 110° ermöglichen.

6. Strahlenschutzvorrichtung (10) nach einem der Ansprüche 1 bis 5, wobei am Träger (11) ferner mindestens ein Untertischstrahlenschutzelement (13), vorzugsweise mehrere Untertischstrahlenschutzelemente (13) nebeneinander und überlappend, lösbar anbringbar ist, wobei das Untertischstrahlenschutzelement (13) vorzugsweise schwingbar aufgehängt ist.

7. Strahlenschutzvorrichtung (10) nach einem der Ansprüche 1 bis 6, wobei das Abschirmelement (12) abgewinkelt ist.

8. Strahlenschutzvorrichtung (10) nach einem der Ansprüche 1 bis 7, wobei der Träger (11) durch das Abschirmelement (12) in der heruntergeklappten Stellung im Wesentlichen abgedeckt ist.

## Claims

1. A radiation protective device (10) for attachment to a patient table (2), wherein the device comprises a carrier (11) and a shielding element (12) provided at the carrier (11), wherein the shielding element (12) is mounted at the carrier (11) by means of at least one double joint (15) such that the shielding element (12) is movable at least between a folded-up position and a folded-down position; wherein the shielding element (12) is adjustable in height and pivotable and/or inclinable at least in its folded-up position; and wherein the double joint (15) is attached to the carrier (11) such that at least one swivelling axis of the double joint (15) is blocked in the folded-up position.

2. The radiation protective device (10) according to claim 1, wherein the double joint (15) is configured as a double joint pin (15).

3. The radiation protective device (10) according to claim 2, wherein the at least one double joint pin (15) is passed through a through-hole (11a) in the carrier (11).

4. The radiation protective device (10) according to claim 3, wherein the at least one double joint pin (15) comprises three pin portions articulated to each other, wherein a first pin end portion (15a) has a length (L1) which is greater than the thickness (D) of the carrier (11), wherein a central pin portion (15b) preferably has a length (L3) which is greater than the distance (d) of the through-hole from the edge of the carrier, and wherein a second pin end portion (15c) preferably has a length (L2) which is smaller than the thickness (D) of the carrier (11).

5. The radiation protective device (10) according to any one of claims 1 to 4, wherein each of the two joints of the at least one double joint pin (15) permits pivoting angles in the range between 70° and 110°.

6. The radiation protective device (10) according to any one of claims 1 to 5, wherein further at least one undertable radiation protective element (13) is attachable at the carrier (11) in a detachable way and preferably a plurality of undertable radiation protective elements (13) are attachable at the carrier in a detachable way side by side and so as to overlap, wherein the undertable radiation protective element (13) is preferably swingably suspended.

7. The radiation protective device (10) according to any one of claims 1 to 6, wherein the shielding element (12) is angled.

8. The radiation protective device (10) according to any one of claims 1 to 7, wherein the carrier (11) is essentially covered by the shielding element (12) in the folded-down position.

## Revendications

1. Ensemble de protection contre les radiations (10) destiné à être placé sur une table de patient (2), l'ensemble comprenant un support (11) et un élément d'écran de protection (12) prévu sur le support (11), et dans lequel l'élément d'écran de protection (12) est monté sur le support (11) au moyen d'au moins une articulation double (15), de manière telle, que l'élément d'écran de protection (12) soit mobile au moins entre une position basculée vers le haut et une position rabattue vers le bas,
dans lequel l'élément d'écran de protection (12) est, au moins dans la position basculée vers le haut, réglable en hauteur et pivotant et/ou inclinable,
et dans lequel l'articulation double (15) est rapportée sur le support (11) de façon telle, que dans la position basculée vers le haut, au moins un axe de pivotement de l'articulation double (15) soit bloqué.

2. Ensemble de protection contre les radiations (10) selon la revendication 1, dans lequel l'articulation double (15) est réalisée en tant que broche d'articulation double (15).

3. Ensemble de protection contre les radiations (10) selon la revendication 2, dans lequel ladite au moins une broche d'articulation double (15) est menée et guidée à travers une ouverture de passage (11a) dans le support (11).

4. Ensemble de protection contre les radiations (10) selon la revendication 3, dans lequel ladite au moins une broche d'articulation double (15) présente trois tronçons de broche reliés de manière articulée les uns aux autres, dans lequel un premier tronçon d'extrémité de broche (15a) présente une longueur (L1), qui est supérieure à l'épaisseur (D) du support (11), dans lequel un tronçon de broche central (15b) présente de préférence une longueur (L3), qui est supérieure à la distance (d) de l'ouverture de passage au bord du support, et dans lequel un deuxième tronçon d'extrémité de broche (15c) présente de préférence une longueur (L2), qui est inférieure à l'épaisseur (D) du support (11).

5. Ensemble de protection contre les radiations (10) selon l'une des revendications 1 à 4, dans lequel les deux articulations de ladite au moins une articulation double (15) permettent respectivement des angles de pivotement dans une plage entre 70° et 110°.

6. Ensemble de protection contre les radiations (10) selon l'une des revendications 1 à 5, dans lequel sur le support (11) peut par ailleurs être rapporté de manière amovible, au moins un élément de protection contre les radiations de bas de table (13), de préférence plusieurs éléments de protection contre les radiations de bas de table (13) agencés côte à côte et se chevauchant mutuellement, et dans lequel l'élément de protection contre les radiations de bas de table (13) est suspendu de préférence de manière oscillante.

7. Ensemble de protection contre les radiations (10) selon l'une des revendications 1 à 6, dans lequel l'élément d'écran de protection (12) est coudé.

8. Ensemble de protection contre les radiations (10) selon l'une des revendications 1 à 7, dans lequel le support (11) est sensiblement recouvert par l'élément d'écran de protection (12) dans la position rabattue vers le bas.
